(19) Europäisches Patentamt
European Patent Office
Office européen des brevets

(11) Veröffentlichungsnummer: **0 687 467 A2**

# (12) EUROPÄISCHE PATENTANMELDUNG

(21) Anmeldenummer: **95107332.9**

(22) Anmeldetag: **15.05.95**

(51) Int. Cl.[6]: **A61K 31/075**, A61K 31/16, A61K 31/045

(30) Priorität: **14.06.94 DE 4420625**

(43) Veröffentlichungstag der Anmeldung: **20.12.95 Patentblatt 95/51**

(84) Benannte Vertragsstaaten: **AT BE CH DE ES FR GB IT LI NL**

(71) Anmelder: **Beiersdorf Aktiengesellschaft**
**Unnastrasse 48**
**D-20245 Hamburg (DE)**

(72) Erfinder: **Schönrock, Uwe, Dr.**
**Margaritenweg 8**
**D-22844 Norderstedt (DE)**
Erfinder: **Degwert, Joachim, Dr.**
**Am Buchenweg 9**
**D-21255 Tostedt (DE)**
Erfinder: **Steckel, Friedhelm, Dr.**
**Delle 6**
**D-22395 Hamburg (DE)**

## (54) Wirkstoffkombinationen mit einem Gehalt and Glyceylalkylethern und kosmetische und dermatologische Zubereitungen, solche Wirkstoffkombinationen enthaltend

(57) Wirkstoffkombinationen, bestehend aus wirksamen Anteilen
(a) eines oder mehrerer Glycerinether gesättigter und/oder ungesättigter, verzweigter und/oder unverzweigter aliphatischer Alkohole mit 12 bis 24 Kohlenstoffatomen
(b) Bisabolol und/oder Panthenol
(c) sowie gegebenenfalls eines oder mehrerer Stoffe, gewählt aus der Gruppe der kosmetisch oder dermatologisch akzeptablen Antioxidantien.

EP 0 687 467 A2

Rank Xerox (UK) Business Services
(3.10/3.09/3.3.4)

Die vorliegende Erfindung betrifft Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen. Ferner betrifft die Erfindung die Verwendung solcher Wirkstoffe und Zubereitungen, solche Wirkstoffe enthaltend, zur Immunstimulation der Haut, dabei vorteilhaft auch zur Immunstimulation im Sinne einer Behandlung der verletzten Haut, insbesondere zur Behandlung von Wunden.

Darüberhinaus betrifft die Erfindung Zubereitungen mit extrem niedrigem sogenanntem "Stinging Potential".

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismen spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Immunsuppression im allgemeinen ist die Unterdrückung oder Abschwächung der Reaktivität des Immunsystems. Die Immunsuppression kann in lokale und systemische Effekte aufgegliedert werden. Letztlich umfaßt sie eine Vielzahl verschiedenster Aspekte, welche alle eine Reduktion der normalen immunologischen Abwehrmechanismen der Haut beinhalten.

Bei Menschen mit sensibler, empfindlicher oder verletzlicher Haut kann ein mit "Stinging" (〈engl.〉 "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Als neurosensorisches Phänomen ist der Juckreiz bei atopischer Haut anzusehen, sowie Juckreiz bei Hauterkrankungen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluß wie Sonne, Kälte, Trockenheit, aber auch feuchte Wärme, Wärmestrahlung und UV-Strahlung, z.B. der Sonne, hervorgerufen werden.

In "Journal of the Society of Cosmetic Chemists" 28, S.197 - 209 (Mai 1977) beschreiben P.J.Frosch und A.M.Kligman eine Methode zur Abschätzung des "Stinging-Potentials" topisch verabreichter Substanzen. Als positive Substanzen werden hier z.B. Milchsäure und Brenztraubensäure eingesetzt. Bei Messung nach dieser Methode wurden aber auch Aminosäuren, insbesondere Glycin, als neurosensorisch aktiv ermittelt (solche Substanzen werden "Stinger" genannt).

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz "Stingingeffekte" erlebt, wird sie mit hoher Wahrscheinlichkeit bei jedem weiteren Kontakt wiederholt erleben. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Es war also die Aufgabe der vorliegenden Erfindung, den Nachteilen des Standes der Technik abzuhelfen.

Insbesondere sollten Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" Zur Verfügung gestellt werden.

Ferner sollten solche Wirkstoffe, bzw. Zubereitungen, solche Wirkstoffe enthaltend, zur Verfügung gestellt werden, welche zur Immunstimulation der Haut, dabei vorteilhaft auch zur Immunstimulation im Sinne der die Wundheilung fördernden Wirkung, verwendet werden können.

Erstaunlicherweise helfen Wirkstoffkombinationen, bestehend aus wirksamen Anteilen

(a) eines oder mehrerer Glycerinether gesättigter und/oder ungesättigter, verzweigter und/oder unverzweigter aliphatischer Alkohole mit 12 bis 24 Kohlenstoffatomen

(b) Bisabolol und/oder Panthenol

(c) sowie gegebenenfalls eines oder mehrerer Stoffe, gewählt aus der Gruppe der kosmetisch oder dermatologisch akzeptablen Antioxidantien,

den Nachteilen des Standes der Technik ab.

Insbesondere wird die vorliegende Erfindung verkörpert durch die Verwendung von Wirkstoffkombinationen, bestehend aus wirksamen Anteilen

(a) eines oder mehrerer Glycerinether gesättigter und/oder ungesättigter, verzweigter und/oder unverzweigter aliphatischer Alkohole mit 12 bis 24 Kohlenstoffatomen

(b) Bisabolol und/oder Panthenol

(c) sowie gegebenenfalls eines oder mehrerer Stoffe, gewählt aus der Gruppe der kosmetisch oder dermatologisch akzeptablen Antioxidantien,

zur Behandlung und/oder Prophylaxe erythematöser Hauterscheinungen und/oder zur Stimulierung des Immunsystems, insbesondere zur Wundheilung.

Die erfindungsgemäßen Substanzkombinationen wirken synergistisch in bezug auf die jeweiligen Einzelsubstanzen. Dabei entfalten sie antiphlogistische, antierythematöse, immunstimulierende sowie die Wundheilung fördernde Wirkung.

Die Verwendung der vorliegenden Wirkstoffkombinationen oder Zubereitungen zur Prophylaxe oder Behandlung erythematöser Hauterscheinungen oder zur Heilung von Wunden ist vorteilhaft, insbesondere auch die Verwendung solcher Wirkstoffkombinationen zur Herstellung von Zubereitungen zur Prophylaxe oder Behandlung erythematöser Hauterscheinungen oder zur Heilung von Wunden.

Die erfindungsgemäßen Substanzkombination können dabei sowohl in kosmetischen als auch in medizinischen Zubereitungen Verwendung finden.

Zwar ist, die Verwendung der Einzelsubstanzen in Kosmetika bzw. Dermatika durchaus bekannt. So beschreibt die US-PS 3,294,639 eine Methode zur Behandlung von entzündlichen Erscheinungen mit einer wirksamen Menge an Batylalkohol, Chimylalkohol und/oder Selachylalkohol.

Die antientzündliche Wirkung bzw. andere physiologische Wirkung solcher Glycerylether ist darüberhinaus aus wissenschaftlichen Schriften zu entnehmen: R. G. Burford, C. W. Gowdey, Arch. Int. Pharmacodyn., 1968, 173, Nr. 1, S. 56 ff.; J.Bodman, J.H.Maisin, "The α-Glyceryl Ethers", Clinica Chimica Acta Vol.3, 1958, S. 253 ff.

Die erfindungsgemäßen Wirkstoffkombinationen bzw. deren vorteilhaften Eigenschaften sind hingegen nicht aus dem Stande der Technik herleitbar.

Erfindungsgemäß bevorzugt zeichnen sich die Glycerinether durch folgende Struktur aus:

```
HO-CH-CH-C-O-(CH2)l-(CH=CH)m-(CH2)n-CH3
   |  |
   H  OH
```

Dabei nehmen die Indizes l, m, n Werte von 0 bis 23 an, mit der Maßgabe, daß die Summe aus $l + n + (2 \times m)$ Zahlen im Bereich zwischen 11 und 23 ergeben muß.

Vorteilhafte Vertreter der erfindungsgemäßen Glycerinester sind beispielsweise Chimylalkohol

```
HO-CH-CH-C-O-(CH2)15-CH3
   |  |
   H  OH
```

(α-Hexadecylglycerylether),

Selachylalkohol

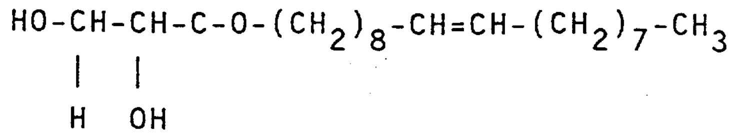

(α-9-Octadecenylglycerylether)
und, ganz besonders bevorzugt,
Batylalkohol

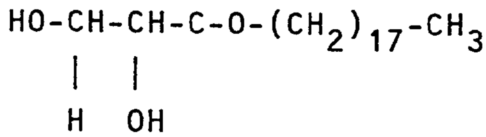

(α-Octadecylglycerylether).

α-Bisabolol, chemisch: [(-)-6-Methyl-2-(4-methyl-3-cyclohexenyl)-5-hepten-2-ol], ist eine im Kamillenöl vorkommendeSubstanz mit antiphlogistischer und spasmolytischer Wirkung. Es wird sowohl auf dem Gebiete der Kosmetik (beispielsweise als Parfümfixateur) als auch in der Medizin genutzt. Es ist durch die Strukturformel

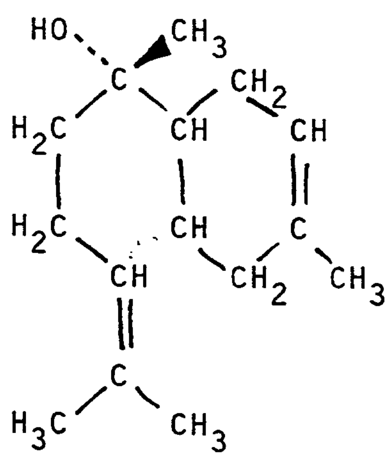

gekennzeichnet.

Panthenol, chemisch: 2,4-Dihydroxy-N-(3-hydroxypropyl)-3,3-dimethylbutyramid, wird bekanntermaßen auf dem Gebiete der Komsetik , aber auch zur Behandlung von Entzündungen eingesetzt. Es ist durch die Strukturformel

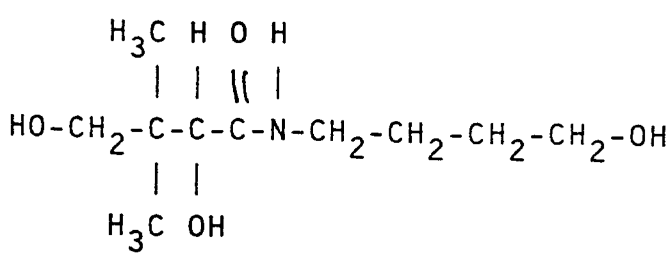

gekennzeichnet.

Erfindungsgemäß können als günstige, aber dennoch fakultativ zu verwendende Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus

Ascorbinsäure (Vitamin C), Ascorbinsäurederivaten, den verschiedenen Tocopherolen (Vitamin E) und Tocopherylestern bzw. anderen Tocopherolderivaten, Folsäure (früher als Vitamin $B_c$, $B_9$, oder M bezeichnet, heute der Vitamin $B_2$-Gruppe zugeordnet), Phytinsäure (Inosithexaphosphorsäure, auch Fytinsäure), den verschiedenen Ubichinonen (Mitochinone, Coenzyme Q), Gallenextrakt, cis- und/oder trans-Urocaninsäure (4-Imidazolylacrylsäure), Carnosin (N-$\beta$-Alanyl-L-histidin, Ignotin), Histidin, Flavonen oder Flavonoiden, Cystin (3,3'-Dithiobis(2-aminopropionsäure), Cystein (2-Amino-3-mercaptopropionsäure) und dessen Derivaten (z.B. N-Acetylcystein), die verschiedenen Carotine (insbesondere $\beta$-Carotin und Lycopin (Psi-Carotin)), Tyrosin (2-Amino-3-(4-Hydroxyphenyl)-propionsäure), $\alpha$-Liponsäure (1,2-Dithiolan-3-pentansäure) sowie Glutathion (gamma-L-Glutamyl-L-cysteinglycin) und Glutathionester.

Es ist von Vorteil, das Verhältnis der Glycerinalkylether zu Bisabolol und/oder Panthenol aus dem Bereich von 1 : 20 bis 20 : 1, bevorzugt 1 : 5 bis 5 : 1, insbesondere 2 : 3 bis 3 : 2, zu wählen.

Liegen mehrere Glycerinalkylether vor, oder werden Bisabolol und Panthenol gleichzeitig eingesetzt, so wird das Verhältnis der Gesamtmenge der Glycerinalkylether zur Gesamtmenge aus Bisabolol und Panthenol vorteilhaft gewählt aus dem Bereich von 1 : 20 bis 20 : 1, bevorzugt 1 : 5 bis 5 : 1, insbesondere 2 : 3 bis 3 : 2.

Weiterhin ist von Vorteil, das Verhältnis der Glycerinalkylether zu Bisabolol und/oder Panthenol zu Antioxidantien aus dem Bereich von 1 : 1 : 20 bis 20 : 20 : 1, bevorzugt 1 : 1 : 5 bis 5 : 5 : 1.

Liegen mehrere Glycerinalkylether bzw. Antioxidantien vor, oder werden Bisabolol und Panthenol gleichzeitig eingesetzt, so wird das Verhältnis der Gesamtmenge der Glycerinalkylether zur Gesamtmenge aus Bisabolol und Panthenol zur Gesamtmenge der Antioxidantien vorteilhaft gewählt aus dem Bereich von 1 : 1 : 20 bis 20 : 20 : 1, bevorzugt 1 : 1 : 5 bis 5 : 5 : 1.

In kosmetischen oder dermatologischen Zubereitungen betragen die Einzelkonzentrationen der erfindungsgemäßen Wirkstoffkombinationen vorzugsweise

0,01 - 10 Gew.-% eines oder mehrerer Glycerinether gesättigter und/oder ungesättigter, verzweigter und/oder unverzweigter aliphatischer Alkohole mit 12 bis 24 Kohlenstoffatomen

0,5 - 5 Gew.-% Bisabolol und/oder Panthenol

0,1 - 2,5 Gew.-% eines oder mehrerer Stoffe, gewählt aus der Gruppe der kosmetisch oder dermatologisch akzeptablen Antioxidantien,

jeweils bezogen auf das Gesamtgewicht der kosmetischen oder dermatologischen Zubereitungen.

Sofern Gallenextrakt und/oder $\alpha$-Liponsäure als erfindungsgemäße Antioxidantien Verwendung finden, ist vorteilhaft, deren jeweilige Konzentrationen aus dem Bereich von 0,001 - 2 Gew.-%, bezogen auf das Gesamtgewicht der Formulierung, zu wählen.

Sofern Vitamin E und/oder dessen Derivate als erfindungsgemäße Antioxidantien Verwendung finden, ist vorteilhaft, deren jeweilige Konzentrationen aus dem Bereich von 0,001 - 4 Gew.-%, bezogen auf das Gesamtgewicht der Formulierung, zu wählen.

Wenn Retinoide, Carotine und/oder deren Derivate und/oder Lycopin als erfindungsgemäße Antioxidantien Verwendung finden, ist vorteilhaft, deren jeweilige Konzentrationen aus dem Bereich von 0,001 - 10 Gew.-%, bezogen auf das Gesamtgewicht der Formulierung, zu wählen.

Sofern Urocaninsäure als erfindungsgemäßes Antioxidans Verwendung findet, ist vorteilhaft, ihre Konzentration aus dem Bereich von 0,001 - 2,00 Gew.-%, bezogen auf das Gesamtgewicht der Formulierung, zu wählen.

Es ist dem Fachmanne natürliche bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, die erfindungsgemäßen Wirkstoffkombination als Zusatzstoff zu Zubereitungen zu geben, die bereits andere Wirkstoffe für andere Zwecke enthalten.

Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Insbesondere können die erfindungsgemäßen Wirkstoffkombinationen in als Zusatzstoff in kosmetischen Desodorantien oder Antitranspirantien verwendet werden. Als desodorierend bzw. antitranspirierend wirksame Agentien können dann die üblichen, dem Fachmanne bekannten Substanzen verwendet werden. Beispielsweise kann durch Adstringentien - vorwiegend Aluminiumsalze wie Aluminiumhydroxychlorid (Aluchlorhydrat) - die Entstehung des Schweißes unterbunden werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Vorteilhaft sind beispielsweise Monocarbonsäureester des Di- bzw. Triglycerins. Aber auch andere antimikrobiell wirksame Stoffe sind geeignet.

Erfindungsgemäß ist sogar die Verwendung an sich nicht sonderlich milder Wirkstoffe möglich und gegebenenfalls vorteilhaft, da deren eventuelle erythemfördernde Wirkung von den erfindungsgemäßen Wirkstoffkombinationen kompensiert werden können.

Auch die Verwendung der Wirkstoffkombinationen in hautpflegenden kosmetischen und/oder dermatologischen Zubereitungen wird als vorteilhafte Verkörperung der vorliegenden Erfindung angesehen.

Zur Anwendung werden die erfindungsgemäßen kosmetischen und dermatologischen Zubereitungen in der für Kosmetika üblichen Weise kauf die Haut in ausreichender Menge aufgebracht.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen außerdem Substanzen enthalten, die UVStrahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:

- 3-Benzylidencampher-Derivate, vorzugsweise
  3-(4-Methylbenzyliden)campher, 3-Benzylidencampher;
- 4-Aminobenzoesäure-Derivate, vorzugsweise
  4-(Dimethylamino)-benzoesäure(2-ethylhexyl)ester,
  4-(Dimethylamino)benzoesäureamylester;
- Ester der Zimtsäure, vorzugsweise
  4-Methoxyzimtsäure(2-ethylhexyl)ester,
  4-Methoxyzimtsäureisopentylester;
- Ester der Salicylsäure, vorzugsweise
  Salicylsäure(2-ethylhexyl)ester,
  Salicylsäure(4-isopropylbenzyl)ester,
  Salicylsäurehomomenthylester;
- Derivate des Benzophenons, vorzugsweise
  2-Hydroxy-4-methoxybenzophenon,
  2-Hydroxy-4-methoxy-4'-methylbenzophenon,
  2,2'-Dihydroxy-4-methoxybenzophenon;
- Ester der Benzalmalonsäure, vorzugsweise
  4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)-benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, die erfindungsgemäßen Wirkstoffkombinationen mit UVA-Filtern zu kombinieren, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)-propan-1,3-dion. Auch diese Kombinationen bzw. Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen, enthaltend die erfindungsgemäßen Wirkstoffkombinationen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Erfindungsgemäße kosmetische und dermatologische Zubereitungen zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Erfindungsgemäße Emulsionen, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Emulsion darstellt, können als Lösungsmittel verwendet werden:

- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäße Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Erfindungsgemäße feste Stifte enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische und/oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bei erfindungsgemäßen kosmetischen und dermatologischen Zubereitungen für die Verwendung am Haar handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und/oder dermatologischen Zubereitungen enthalten gegebenenfalls zusätzliche Wirkstoffe, Hilfs- und/oder Zusatzstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte sowie zusätzliche Substanzen zum Rückfetten der Haare bzw. der Kopfhaut.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, eine wirksame Menge an erfindungsgemäßen Wirkstoffkombinationen, und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wir, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen und/oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie die erfindungsgemäßen Wirkstoffkombinationen. Die Menge der verwendeten Polymeren liegt z.B. zwischen 0,1 und 10 Gew.-%, bevorzugt zwischen 0,1 und 3 Gew.-%.

Erfindungsgemäße kosmetische Zubereitungen zur Behandlung und Pflege der Haare, die erfindungsgemäße Wirkstoffkombinationen enthalten, können als Emulsionen vorliegen, dies vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Kosmetische Zubereitungen zur Behandlung und Pflege der Haare können als Gele vorliegen, die neben einem wirksamen Gehalt an den erfindungsgemäßen Wirkstoffkombinationen und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdikkungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine

färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die nachfolgenden Beispiele sollen die vorliegende Erfindung verdeutlichen.

**Beispiel 1**

| Salbe, W/O-Emulsion | |
|---|---|
| (a) | Gew.-% |
| Mikrokristallines Wachs + Pentaerythritylcocoat + Stearylcitrat + Glyceryloleat + Aluminiumstearat + Propylenglycol (Dehymuls F, Henkel) | 8,00 |
| Decyloleat (Cetiol V, Henkel) | 7,00 |
| Petrolatum (Vaseline BD, Witco) | 20,00 |
| Paraffinöl | 12,00 |
| Mikrokristallines Wachs | 2,00 |
| (b) | |
| Olivenöl | 12,00 |
| Batylalkohol | 2,50 |
| (c) | |
| Panthenol (Roche) | 2,50 |
| Konservierungsmittel | q.s. |
| Parfüm | q.s. |
| (d) | |
| Glycerin | 3,00 |
| $MgSO_4$ | 0,70 |
| Wasser | ad 100,00 |

Herstellung: Die Phasen (a), (b) und (d) werden getrennt auf 75° C erwärmt. Sodann werden die Phasen (a) und (b) gleichmäßig in die Phase (d) eingerührt. Hernach wird auf ca. 65° C abgekühlt, homogenisiert und dann auf ca. 40° C abgekühlt. Nacheinander werden das Panthenol sowie gewünschtenfalls geeignete Mengen an Konservierungsmittel und/oder Parfüm in das entstandene Gemisch eingebracht.

Dann wird das Gemisch auf 30° C abgekühlt und noch einmal homogenisiert.

**Beispiel 2**

| Crème, O/W-Emulsion | |
|---|---|
| (a) | Gew.-% |
| Glycerylstearat SE (Tegin Normal, Th.Goldschmidt)<br>Ceteareth 20 (Eumulgin B2, Henkel)<br>Petrolatum (Vaseline BD, Witco)<br>Paraffinöl<br>Cetearylalkohl (Lanette O, Henkel) | 1,00<br>1,00<br>1,00<br>14,00<br>1,50 |
| (b) | |
| Propylenglycol<br>Batylalkohol | 5,00<br>2,50 |
| (c) | |
| D-Panthenol 75 L (Roche)<br>Carbomer (Synthalen M, Sigma Chemie)<br>Wasser<br>Konservierungsmittel | 2,50<br>0,80<br>20,00<br>q.s. |
| (d) | |
| Parfüm | q.s. |
| (e) | |
| NaOH (45 %)<br>Wasser | 0,60<br>ad 100,00 |

Herstellung: Die Phasen (a), (b) und (e) werden getrennt auf 75° C erwärmt. Sodann werden die Phasen (a) und (b) gleichmäßig in die Phase (e) eingerührt. Hernach wird auf ca. 65° C abgekühlt, homogenisiert und dann auf ca. 40° C abgekühlt. Nacheinander werden die Phasen (c) und gewünschtenfalls eine geeignete Menge an Parfüm in das entstandene Gemisch eingebracht. Dann wird das Gemisch auf 30° C abgekühlt und noch einmal homogenisiert.

**Beispiel 3**

| Lotion, W/O-Emulsion | |
|---|---|
| (a) | Gew.-% |
| Paraffinöl<br>Aluminiumstearat | 9,50<br>0,35 |
| (b) | |
| PEG-40-Sorbitanperoleat (Arlatone T, Henkel)<br>Polyglycerin-3-diisostearat (Lameform TGI, Henkel)<br>Petrolatum (Vaseline BD, Witco)<br>Cetylpalmitat (Cutina CP, Henkel) | 2,00<br>2,00<br>1,70<br>1,50 |
| (c) | |
| Isohexadecan (Solvent IH) | 6,00 |
| (d) | |
| Propylenglycol<br>Batylalkohol | 5,00<br>2,50 |
| (e) | |
| D-Panthenol 75 L (Roche)<br>Konservierungsmittel<br>Parfüm | 2,50<br>q.s.<br>q.s. |
| (f) | |
| $MgSO_4$<br>Wasser | 0,60<br>ad 100,00 |

Herstellung: Phase (a) wird auf 120° C erhitzt, dann auf 80° C abgekühlt. Die Phasen (b) und (d) werden getrennt auf 80° C erwärmt. Sodann werden die Phasen (b) und (d) gleichmäßig in die Phase (a) eingerührt. Die vereinigten Phasen (a), (b) und (d) werden zu der auf 75° C erhitzten Phase (f) gegeben, hernach wird auf 65° C abgekühlt und homogenisiert. Phase (c) wird hinzugegeben, es wird auf 40° C abgekühlt. Die Bestandteile (e) werden eingerührt, es wird auf 30° C abgekühlt und erneut homogenisiert.

**Beispiel 4**

| Lotion, O/W-Emulsion | |
|---|---|
| (a) | Gew.-% |
| Acrylat/$C_{10-30}$-Alkylacrylatcopolymer (Pemulen TR 1, Goodrich)<br>Cetearylalkohol (Lanette O, Henkel)<br>$C_{12-15}$ Alkylbenzoate (Finsolv TN, Erbslöh) | 0,30<br>2,00<br>3,00 |
| (b) | |
| D-Panthenol 75 L (Roche) | 2,50 |
| (c) | |
| Ethanol<br>Batylalkohol | 5,00<br>2,50 |
| (d) | |
| Konservierungsmittel<br>Parfüm | q.s.<br>q.s. |
| (e) | |
| Butandiol 1,3<br>NaOH (45 %)<br>Wasser | 10,00<br>0,22<br>ad 100,00 |

Herstellung: Phase (a) und e) werden auf 75° C erhitzt, dann auf 65° C abgekühlt. Die vereinigten Phasen werden homogenisiert und auf 40° C abgekühlt. Nacheinander werden Panthenol, Phase (c) und gegebenenfalls die Bestandteile (d) eingerührt. Es wird auf 30° C abgekühlt und erneut homogenisiert.

**Beispiel 5**

| Gel | |
|---|---|
| (a) | Gew.-% |
| Propylenglycol 1,2<br>Batylalkohol | 5,00<br>2,50 |
| (b) | |
| Bisabolol | 2,50 |
| (c) | |
| Carbomer (Carbopol 2984) | 0,80 |
| (d) | |
| NaOH (45 %) | 0,70 |
| (e) | |
| Konservierungsmittel<br>Parfüm | q.s.<br>q.s. |
| (f) | |
| PEG-150 (Polywachs 6000S, Hüls)<br>Sorbitol<br>Wasser | 4,00<br>0,50<br>ad 100,00 |

Herstellung: Die Phasen (a) und (f) werden getrennt auf 75° C erhitzt und zusammengerührt. In die vereinigten Phasen wird das Bisabolobol gleichmäßig eingerührt, sodann das Carbomer eingerührt, das Gemisch wird auf 65° C abgekühlt und homogenisiert. Nach Neutralisieren mit NaOH wird auf 40° C abgekühlt. Die Bestandteile (e) werden gewünschtenfalls zugegeben, sodann wird erneut homogenisiert.

**Beispiel 6**

| Salbe, W/O-Emulsion | |
|---|---|
| (a) | Gew.-% |
| Mikrokristallines Wachs + Pentaerythritylcocoat + Stearylcitrat + Glyceryloleat + Aluminiumstearat + Propylenglycol (Dehymuls F, Henkel) | 8,00 |
| Decyloleat (Cetiol V, Henkel) | 7,00 |
| Petrolatum (Vaseline BD, Witco) | 20,00 |
| Paraffinöl | 12,00 |
| Mikrokristallines Wachs | 2,00 |
| $\alpha$-Tocopherylacetat | 0,50 |
| (b) | |
| Olivenöl | 12,00 |
| Batylalkohol | 2,50 |
| (c) | |
| Panthenol (Roche) | 2,50 |
| Konservierungsmittel | q.s. |
| Parfüm | q.s. |
| (d) | |
| Glycerin | 3,00 |
| $MgSO_4$ | 0,70 |
| Wasser | ad 100,00 |

Herstellung: Die Phasen (a), (b) und (d) werden getrennt auf 75° C erwärmt. Sodann werden die Phasen (a) und (b) gleichmäßig in die Phase (d) eingerührt. Hernach wird auf ca. 65° C abgekühlt, homogenisiert und dann auf ca. 40° C abgekühlt. Nacheinander werden das Panthenol sowie gewünschtenfalls geeignete Mengen an Konservierungsmittel und/oder Parfüm in das entstandene Gemisch eingebracht. Dann wird das Gemisch auf 30° C abgekühlt und noch einmal homogenisiert.

**Beispiel 7**

| Crème, O/W-Emulsion | |
|---|---|
| (a) | Gew.-% |
| Glycerylstearat SE (Tegin Normal, Th.Goldschmidt)<br>Ceteareth 20 (Eumulgin B2, Henkel)<br>Petrolatum (Vaseline BD, Witco)<br>Paraffinöl<br>Cetearylalkohl (Lanette O, Henkel)<br>$\alpha$-Tocopherylacetat | 1,00<br>1,00<br>1,00<br>14,00<br>1,50<br>0,50 |
| (b) | |
| Propylenglycol<br>Batylalkohol | 5,00<br>2,50 |
| (c) | |
| D-Panthenol 75 L (Roche)<br>Carbomer (Synthalen M, Sigma Chemie)<br>Wasser<br>Konservierungsmittel | 2,50<br>0,80<br>20,00<br>q.s. |
| (d) | |
| Parfüm | q.s. |
| (e) | |
| NaOH (45 %)<br>Wasser | 0,60<br>ad 100,00 |

Herstellung: Die Phasen (a), (b) und (e) werden getrennt auf 75° C erwärmt. Sodann werden die Phasen (a) und (b) gleichmäßig in die Phase (e) eingerührt. Hernach wird auf ca. 65° C abgekühlt, homogenisiert und dann auf ca. 40° C abgekühlt. Nacheinander werden die Phasen (c) und gewünschtenfalls eine geeignete Menge an Parfüm in das entstandene Gemisch eingebracht. Dann wird das Gemisch auf 30° C abgekühlt und noch einmal homogenisiert.

**Beispiel 8**

| Lotion, W/O-Emulsion | |
|---|---|
| (a) | Gew.-% |
| Paraffinöl<br>Aluminiumstearat | 9,50<br>0,35 |
| (b) | |
| PEG-40-Sorbitanperoleat (Arlatone T, Henkel)<br>Polyglycerin-3-diisostearat (Lameform TGI, Henkel)<br>Petrolatum (Vaseline BD, Witco)<br>Cetylpalmitat (Cutina CP, Henkel)<br>$\alpha$-Tocopherylacetat | 2,00<br>2,00<br>1,70<br>1,50<br>0,50 |
| (c) | |
| Isohexadecan (Solvent IH) | 6,00 |
| (d) | |
| Propylenglycol<br>Batylalkohol | 5,00<br>2,50 |
| (e) | |
| D-Panthenol 75 L (Roche)<br>Konservierungsmittel<br>Parfüm | 2,50<br>q.s.<br>q.s. |
| (f) | |
| $MgSO_4$<br>Wasser | 0,60<br>ad 100,00 |

Herstellung: Phase (a) wird auf 120° C erhitzt, dann auf 80° C abgekühlt. Die Phasen (b) und (d) werden getrennt auf 80° C erwärmt. Sodann werden die Phasen (b) und (d) gleichmäßig in die Phase (a) eingerührt. Die vereinigten Phasen (a), (b) und (d) werden zu der auf 75° C erhitzten Phase (f) gegeben, hernach wird auf 65° C abgekühlt und homogenisiert. Phase (c) wird hinzugegeben, es wird auf 40° C abgekühlt. Die Bestandteile (e) werden eingerührt, es wird auf 30° C abgekühlt und erneut homogenisiert.

**Beispiel 9**

| Lotion, O/W-Emulsion | |
|---|---|
| (a) | Gew.-% |
| Acrylat/$C_{10-30}$-Alkylacrylatcopolymer (Pemulen TR 1, Goodrich)<br>Cetearylalkohol (Lanette O, Henkel)<br>$C_{12-15}$ Alkylbenzoate (Finsolv TN, Erbslöh)<br>$\alpha$-Tocopherylacetat | 0,30<br>2,00<br>3,00<br>0,50 |
| (b) | |
| D-Panthenol 75 L (Roche) | 2,50 |
| (c) | |
| Ethanol<br>Batylalkohol | 5,00<br>2,50 |
| (d) | |
| Konservierungsmittel<br>Parfüm | q.s.<br>q.s. |
| (e) | |
| Butandiol 1,3<br>NaOH (45 %)<br>Wasser | 10,00<br>0,22<br>ad 100,00 |

Herstellung: Phase (a) und e) werden auf 75° C erhitzt, dann auf 65° C abgekühlt. Die vereinigten Phasen werden homogenisiert und auf 40° C abgekühlt. Nacheinander werden Panthenol, Phase (c) und gegebenenfalls die Bestandteile (d) eingerührt. Es wird auf 30° C abgekühlt und erneut homogenisiert.

**Beispiel 10**

| Gel | |
|---|---|
| (a) | Gew.-% |
| Propylenglycol 1,2<br>Batylalkohol<br>α-Tocopherylacetat | 5,00<br>2,50<br>0,50 |
| (b) | |
| Bisabolol | 2,50 |
| (c) | |
| Carbomer (Carbopol 2984) | 0,80 |
| (d) | |
| NaOH (45 %) | 0,70 |
| (e) | |
| Konservierungsmittel<br>Parfüm | q.s.<br>q.s. |
| (f) | |
| PEG-150 (Polywachs 6000S, Hüls)<br>Sorbitol<br>Wasser | 4,00<br>0,50<br>ad 100,00 |

Herstellung: Die Phasen (a) und (f) werden getrennt auf 75° C erhitzt und zusammengerührt. In die vereinigten Phasen wird das Bisabolobol gleichmäßig eingerührt, sodann das Carbomer eingerührt, das Gemisch wird auf 65° C abgekühlt und homogenisiert. Nach Neutralisieren mit NaOH wird auf 40° C abgekühlt. Die Bestandteile (e) werden gewünschtenfalls zugegeben, sodann wird erneut homogenisiert.

**Beispiel 11**

| Deocrème, O/W-Emulsion | |
|---|---|
| (a) | Gew.-% |
| Bisabolol<br>Mikrokristallines Wachs + Pentaerythritylcocoat + Stearylcitrat + Glyceryloleat + Aluminiumstearat + Propylenglycol (Dehymuls F, Henkel)<br>Decyloleat (Cetiol V, Henkel)<br>Octyldodecanol (Eutanol G, Henkel)<br>Petrolatum (Vaseline BD, Witco) | 2,50<br>6,00<br><br>7,00<br>5,00<br>5,00 |
| (b) | |
| Batylalkohol<br>Ethanol | 2,50<br>5,00 |
| (c) | |
| Konservierungsmittel<br>Parfüm | q.s.<br>q.s. |
| (d) | |
| Aluminiumchlorhydrat (Locron P, Hoechst)<br>Wasser | 20,00<br>ad 100,00 |

Herstellung: Die Phasen (a) und (b) werden getrennt auf 75° C erhitzt, zur ebenfalls auf 75° C erhitzten Phase (d) gegeben und auf 65° C abgekühlt. Nach Homogenisieren wird auf 40° C abgekühlt. Die Bestandteile (c) werden gewünschtenfalls zugegeben, hernach wird auf 30° C abgekühlt und erneut homogenisiert.

**Beispiel 12**

| Deocrème, O/W-Emulsion | |
|---|---|
| (a) | Gew.-% |
| Bisabolol<br>Mikrokristallines Wachs + Pentaerythritylcocoat + Stearylcitrat + Glyceryloleat + Aluminiumstearat + Propylenglycol (Dehymuls F, Henkel)<br>Decyloleat (Cetiol V, Henkel)<br>Octyldodecanol (Eutanol G, Henkel)<br>Petrolatum (Vaseline BD, Witco)<br>$\alpha$-Tocopherylacetat | 2,50<br>6,00<br><br>7,00<br>5,00<br>5,00<br>0,50 |
| (b) | |
| Batylalkohol<br>Ethanol | 2,50<br>5,00 |
| (c) | |
| Konservierungsmittel<br>Parfüm | q.s.<br>q.s. |
| (d) | |
| Aluminiumchlorhydrat (Locron P, Hoechst)<br>Wasser | 20,00<br>ad 100,00 |

Herstellung: Die Phasen (a) und (b) werden getrennt auf 75° C erhitzt, zur ebenfalls auf 75° C erhitzten Phase (d) gegeben und auf 65° C abgekühlt. Nach Homogenisieren wird auf 40° C abgekühlt. Die Bestandteile (c) werden gewünschtenfalls zugegeben, hernach wird auf 30° C abgekühlt und erneut homogenisiert.

## Patentansprüche

**1.** Wirkstoffkombinationen, bestehend aus wirksamen Anteilen
(a) eines oder mehrerer Glycerinether gesättigter und/oder ungesättigter, verzweigter und/oder unverzweigter aliphatischer Alkohole mit 12 bis 24 Kohlenstoffatomen
(b) Bisabolol und/oder Panthenol
(c) sowie gegebenenfalls eines oder mehrerer Stoffe, gewählt aus der Gruppe der kosmetisch oder dermatologisch akzeptablen Antioxidantien.

**2.** Wirkstoffkombinationen nach Anspruch 1, dadurch gekennzeichnet, daß die Glycerinether die Struktur

$$\begin{array}{l} HO\text{-}CH\text{-}CH\text{-}C\text{-}O\text{-}(CH_2)_l\text{-}(CH{=}CH)_m\text{-}(CH_2)_n\text{-}CH_3 \\ \qquad\;\; | \qquad | \\ \qquad\;\; H \quad\;\; OH \end{array}$$

besitzen, wobei die Indizes l, m, n Werte von 0 bis 23 annehmen, mit der Maßgabe daß die Summe aus l + n + (2 x m) Zahlen im Bereich zwischen 11 und 23 ergeben muß.

**3.** Wirkstoffkombinationen nach Anspruch 2, dadurch gekennzeichnet, daß die Glycerinether gewählt werden aus der Gruppe Chimylalkohol, Selachylalkohol, Batylalkohol.

**4.** Wirkstoffkombinationen nach Anspruch 1, dadurch gekennzeichnet, daß das Verhältnis der Gesamtmenge der Glycerinalkylether zur Gesamtmenge aus Bisabolol und Panthenol gewählt wird aus dem Bereich von 1 : 20 bis 20 : 1, bevorzugt 1 : 5 bis 5 : 1, insbesondere 2 : 3 bis 3 : 2.

**5.** Wirkstoffkombinationen nach Anspruch 1, dadurch gekennzeichnet, daß das Verhältnis der Gesamtmenge der Glycerinalkylether zur Gesamtmenge aus Bisabolol und Panthenol zur Gesamtmenge der Antioxidantien gewählt wird aus dem Bereich von 1 : 1 : 20 bis 20 : 20 : 1, bevorzugt 1 : 1 : 5 bis 5 : 5 : 1.

**6.** Kosmetische oder dermatologische Zubereitungen mit einem Gehalt an Wirkstoffkombinationen nach einem der vorhergehenden Ansprüche.

**7.** Zubereitungen nach Anspruch 8, dadurch gekennzeichnet, daß sie
0,01 - 10 Gew.-% eines oder mehrerer Glycerinether gesättigter und/oder ungesättigter, verzweigter und/oder unverzweigter aliphatischer Alkohole mit 12 bis 24 Kohlenstoffatomen
0,5 - 5 Gew.-% Bisabolol und/oder Panthenol
0,1 - 2,5 Gew.-% eines oder mehrerer Stoffe, gewählt aus der Gruppe der kosmetisch oder dermatologisch akzeptablen Antioxidantien
enthalten.

**8.** Verwendung von Wirkstoffkombinationen oder Zubereitungen nach einem der vorhergehenden Ansprüche zur Prophylaxe oder Behandlung erythematöser Hauterscheinungen oder zur Heilung von Wunden.

**9.** Verwendung von Wirkstoffkombinationen gemäß einem der vorhergehenden Ansprüche zur Herstellung eines Medikaments oder eines Kosmetikums, welches der Prophylaxe oder der Behandlung erythematöser Hauterscheinungen oder der Heilung von Wunden dient.